# EUROPEAN PATENT APPLICATION

(11) **EP 1 332 758 A1**
(43) Date of publication of application: **06.08.2003**
(21) Application number: 03000700.9
(22) Date of filing: 17.01.2003
(51) Int. Cl.: A61K 31/573, A61K 31/4365, A61P 17/00, A61P 17/06

(54) **Dermatological compositions comprising a corticosteroid and zinc pyrithione**

(30) Priority: 17.01.2002 IT BO20020022
(71) Applicant: Rifkin, Bruce, Hawley, Pennsylvania 18428 (US)
(72) Inventor: Sarti, Gianfranco, 61100 Pesaro (IT)
(74) Representative: Modiano, Guido, Dr.-Ing.

(57) **Abstract**

A formulation for treating skin disorders, comprising a composite active ingredient that comprises a) a corticosteroid component and b) zinc pyrithione.

## Description

The present invention relates to pharmaceutical compositions for skin care and to their use to produce skin care drugs.

Creams and ointments for skin care based on a series of monocomponent corticosteroids are currently known.

Said known creams and ointments are not free from drawbacks, including the need for prolonged use and low effectiveness, being therefore costly. Moreover, the presence of tar in some of these products makes them awkward to use. Besides, the low fluidity of these products makes them difficult to apply to large surfaces and to the scalp. Moreover, some known products contain rather aggressive corticosteroids, such as clobetasol, and therefore can become harmful.

The aim of the present invention is to eliminate the drawbacks noted above in known types of pharmaceutical formulation for skin care, providing pharmaceutical formulations that do not require prolonged use, are more effective than known products, are less aggressive and easier to administer even on large surfaces and on the scalp.

An object of the present invention is to provide pharmaceutical compositions for skin care that are less aggressive on the skin and are less likely to have unwanted side effects.

This aim and this and other objects that will become better apparent from the detailed description of the invention that follows are achieved by the pharmaceutical compositions for skin care according to the present invention, which comprise a composite active ingredient which contains a) a corticosteroid component and b) zinc pyrithione.

Preferably, the corticosteroid component is constituted by one or more components selected from the group consisting of augmented betamethasone dipropionate, betamethasone valerate, diflorasone diacetate, diflucortolone valerate, betamethasone dipropionate, hydrocortisone 17-butyrate, fluticasone valerate, halobetasol propionate, momethasone furoate, deoxymethasone, fluocinonide.

The active ingredient, furthermore, can contain coal tar, salicylic acid, selenium sulfate and sulfur.

The pharmaceutical compositions according to the present invention can also include a surfactant that assists in emulsification-suspension, a carrier and an antifungal agent.

In a preferred embodiment, the present invention provides a formulation for treating skin disorders that essentially consists of:
A) 0.01% to 2% by weight of active ingredient;
B) 0.1% to approximately 2% by weight of compound anionic surfactant, selected from the group consisting of of soap, alkyl aryl sulfonate, alkyl ether sulfate, alkyl sulfate and alcohol sulfate;
C) 0.3% to approximately 10% by weight of undecyclenic acid.
D) a composite carrier for balancing said formulation, which contains a first component and a second component in a 1:1 weight ratio, characterized in that the first component is selected from the group consisting of of ethyl alcohol and isopropyl alcohol, and the second component is selected from the group consisting of of isopropyl myristate, isopropyl palmitate, octyl palmitate, octyl isononanoate and isocetyl stearate.

Preferred embodiments of the present invention are:
1) a formulation in which the first component of the carrier is ethyl alcohol and the second component of the carrier is isopropyl myristate,
2) a formulation in which the first component of the carrier is isopropyl alcohol and the second component of the carrier is isopropyl myristate,
3) a formulation that contains approximately 0.05% by weight of betamethasone dipropionate and approximately 0.25% by weight of zinc pyrithione,
4) a formulation that contains approximately 0.1% by weight of sodium lauryl sulfate and 0.3% by weight of undecyclenic acid, and
5) a formulation that contains approximately 0.05% by weight of betamethasone dipropionate, approximately 0.25% by weight of zinc pyrithione, and approximately 0.1% by weight of sodium lauryl sulfate and 0.3% by weight of undecyclenic acid.

In a second aspect, the present invention relates to a formulation for treating skin disorders, which essentially consists of:
a) 0.01% to 0.10% by weight of betamethasone dipropionate;
b) 0.1% to approximately 2% by weight of a compound anionic surfactant, selected from the group consisting of of soap, alkyl aryl sulfonate, alkyl ether sulfate, alkyl sulfate and alcohol sulfate;
c) 0.3% to approximately 10% by weight of undecyclenic acid; and
d) a composite carrier for balancing the formulation, which comprises a first component and a second component in a 1:1 weight ratio, the first component being selected from the group consisting of ethyl alcohol and isopropyl alcohol and the second component being selected from the group consisting of isopropyl myristate, isopropyl palmitate, octyl palmitate, octyl isononanoate and isoacetyl stearate.

Preferred embodiments are constituted by the following formulations:
1) a formulation in which the betamethasone dipropionate content is approximately 0.05% by weight;
2) a formulation in which the anionic surfactant content is approximately 0.1% by weight and is constituted by sodium lauryl sulfate;
3) a formulation according to the preceding item 2, which includes approximately 0.3% undecyclenic acid;
4) a formulation in which the first component of the carrier is ethyl alcohol; and
5) a formulation in which the first component of the carrier is isopropyl alcohol.

Another aspect of the present invention provides a formulation for treating skin disorders that essentially consists of:
a) 0.01% to 2% by weight of zinc pyrithione
b) 0.1% to approximately 12% of a compound anionic surfactant, selected from the group consisting of of soap, alkyl aryl sulfonate, alkyl ether sulfate, alkyl sulfate, and alcohol sulfate;
c) a composite carrier for balancing the formulation, constituted by two components in a 1:1 weight ratio, characterized in that the first component is chosen from the group that consists of ethyl alcohol and isopropyl alcohol and the second component is isopropyl myristate; and
d) 0.3% to 10% by weight of undecyclenic acid.

Preferred embodiments are constituted by the following formulations:
1) a formulation that includes approximately 0.25% by weight of zinc pyrithione;
2) a formulation that contains, as anionic surfactant, approximately 0.1% by weight of sodium lauryl sulfate;
3) a formulation according to the above item 2, which includes approximately 0.3% undecyclenic acid;
4) a formulation in which the first component of the carrier is ethyl alcohol;
5) a formulation in which the first component of the carrier is isopropyl alcohol.

In another aspect, the present invention provides for the use of said pharmaceutical compositions to produce drugs for the treatment of skin disorders, particularly such as psoriasis and other skin disorders characterized by reddening, itching, peeling, scabs and plaques, eczema, seborrheic dermatitis (dandruff), lichen planus and other correlated disorders, for example nummular dermatitis, pompholix, stasis dermatitis, lichen simplex, scratch dermatitis, neurodermatitis, annular granuloma, contact dermatitis, atypical dermatitis.

The inventors of the present invention have found that formulations that contain in association a corticosteroid (for example betamethasone dipropionate, betamethasone valerate, diflorasone diacetate, diflucortolone valerate, betamethasone dipropionate, hydrocortisone 17-butyrate, fluticasone valerate, halobetasol propionate, momethasone furoate, deoxymethasone, fluocinonide) and zinc pyrithione are highly effective and achieve better skin penetration.

Therefore, with respect to all the other products used in these disorders, these new formulations allow to use a smaller quantity for a smaller number of days. The inventor of the present invention has found in fact that the formulations according to the present invention that contain an active ingredient that includes a corticosteroid and zinc pyrithione allow to achieve the first results after approximately one week of treatment. Accordingly, the skin aggressiveness of the formulations according to the present invention is lower, and so is the possibility of unwanted side effects.

The formulations according to the present invention that do not contain tar are usable more easily and less awkwardly.

In another aspect, the present invention relates to a package that contains a pharmaceutical formulation according to the present invention, the package being provided with a mechanism that allows its administration on large surfaces and with an applicator that facilitates the application of the formulation to the scalp, according to its fluidity.

The disclosures in Italian Patent Application No. BO2002A000022 from which this application claims priority are incorporated herein by reference.

## Claims

1. A formulation for treating skin disorders, comprising a composite active ingredient that comprises a) a corticosteroid component and b) zinc pyrithione.

2. The formulation according to claim 1, **characterized in that** said corticosteroid component is selected from the group consisting of augmented betamethasone dipropionate, betamethasone valerate, diflorasone diacetate, diflucortolone valerate, betamethasone dipropionate, hydrocortisone 17-butyrate, fluticasone valerate, halobetasol propionate, momethasone furoate, deoxymethasone and fluocinonide.

3. The formulation according to one of claims 1 or 2, further containing a surfactant, an antifungal agent and a carrier.

4. The formulation according to one or more of the preceding claims, further containing one or more components selected from the group consisting of coal, salicylic acid, selenium sulfate and sulfur.

5. The formulation according to claim 4, which essentially consists of:
a) 0.01-2% by weight of a composite active ingredient that comprises a compound selected from the group consisting of of augmented betamethasone dipropionate, betamethasone valerate, diflorasone diacetate, diflucortolone valerate, betamethasone dipropionate, hydrocortisone 17-butyrate, fluticasone valerate, halobetasol propionate, momethasone furoate, deoxymethasone and fluocinonide, and zinc pyrithione.
b) 0.1-2% by weight of a compound anionic surfactant, selected from the group consisting of soap, alkyl aryl sulfonate, alkyl ether sulfate, alkyl sulfate and alcohol sulfate;
c) 0.3-10% by weight of undecyclenic acid, and
d) a composite carrier, which balances said formulation and is composed of a first component and a second component in a 1:1 weight ratio, **characterized in that** the first component is selected from the group consisting essentially of ethyl alcohol and isopropyl alcohol, and the second component is selected from the group consisting of of isopropyl myristate, isopropyl palmitate, octyl palmitate, octyl isononanoate and isocetyl stearate.

6. The formulation according to claim 5, **characterized in that** said first component of the carrier is ethyl alcohol and said second component of the carrier is isopropyl myristate.

7. The formulation according to claim 5, **characterized in that** said first component of the carrier is isopropyl alcohol and said second component of the carrier is isopropyl myristate.

8. The formulation according to claim 5, containing approximately 0.05% by weight of betamethasone dipropionate and approximately 0.25% by weight of zinc pyrithione.

9. The formulation according to claim 8, containing 0.1% by weight of sodium lauryl sulfate as anionic surfactant and 0.3% by weight of undecyclenic acid.

10. The formulation according to claim 5, comprising 0.1% by weight of sodium lauryl sulfate as anionic surfactant and 0.3% by weight of undecyclenic acid.

11. The formulation according to claim 10, containing 0.05% by weight of betamethasone dipropionate and 0.25% by weight of zinc pyrithione.

12. A formulation for treating skin disorders, which essentially consists of:
a) 0.01-0.10% by weight of betamethasone dipropionate;
b) 0.1-2% by weight of a compound anionic surfactant, selected from the group consisting of soap, alkyl aryl sulfonate, alkyl ether sulfate, alkyl sulfate and alcohol sulfate;
c) 0.3-10% by weight of undecyclenic acid;
d) a composite carrier, which balances the formulation and is constituted by a first component and a second component in a 1:1 weight ratio, the first component of the carrier being selected from the group consisting essentially of ethyl alcohol and isopropyl alcohol, said second component of the carrier being selected from the group consisting of isopropyl myristate, isopropyl palmitate, octyl palmitate, octyl isononanoate and isocetyl stearate.

13. The formulation according to claim 12, comprising 0.05% by weight of betamethasone dipropionate.

14. A formulation for treating skin disorders, which essentially consists of:
a) 0.01-2% by weight of zinc pyrithione;
b) 0.1-12% by weight of a composite anionic surfactant, chosen from the group consisting of soap, alkyl aryl sulfonate, alkyl ether sulfate, alkyl sulfate, and alcohol sulfate;
c) 0.3-10% by weight of undecyclenic acid; and
d) a composite carrier that balances the formulation, said carrier being composed of a first component and a second component in a 1:1 weight ratio, said first component of the carrier being selected from the group essentially consisting of ethyl alcohol and isopropyl alcohol, said second component of the carrier being isopropyl myristate.

15. The formulation according to claim 14, comprising approximately 0.25% by weight of zinc pyrithione.

16. The formulation according to claim 12 or 14, comprising 0.1% by weight of sodium lauryl sulfate as anionic surfactant.

17. The formulation according to claim 12 or 14, comprising approximately 0.3% undecyclenic acid.

18. The formulation according to claim 12 or 14, **characterized in that** the first component of the carrier is ethyl alcohol.

19. The formulation according to claim 12 or 14, **characterized in that** the first component of the carrier is isopropyl alcohol.

20. The formulation according to claim 14, further comprising 0.05% betamethasone dipropionate.

21. Use of a formulation according to any one of the preceding claims for manufacturing a medicament for treating skin disorders.

22. The use according to claim 21, wherein the skin disorder is a disorder such as psoriasis or other skin disorders **characterized by** reddening, itching, peeling, scabs and the generation of plaques, eczema, seborrheic dermatitis (dandruff), lichen planus and other correlated disorders, particularly nummular dermatitis, pompholix, stasis dermatitis, lichen simplex, scratch dermatitis, neurodermatitis, annular granuloma, contact dermatitis, atypical dermatitis.
